Europäisches Patentamt

European Patent Office

Office européen des brevets

(19)

(11) Numéro de publication: **0 210 099 B1**

# FASCICULE DE BREVET EUROPEEN

(12)

(45) Date de publication de fascicule du brevet: **15.04.92**

(51) Int. Cl.5: **A61K 31/40**

(21) Numéro de dépôt: **86401442.8**

(22) Date de dépôt: **30.06.86**

(54) Application du N-(1-allyl-2-pyrrolidinylméthyl) 2-méthoxy 4-amino 5-méthylsulfamoyl benzamide dans le traitement de la maladie de Parkinson.

(30) Priorité: **15.07.85 FR 8510802**

(43) Date de publication de la demande:
**28.01.87 Bulletin 87/05**

(45) Mention de la délivrance du brevet:
**15.04.92 Bulletin 92/16**

(84) Etats contractants désignés:
**AT BE CH DE FR GB IT LI LU NL SE**

(56) Documents cités:
**EP-A- 0 047 207**

**NAUNYN-SCHIEDEBERG'S ARCH. PHARMA-COL., vol. 327, no. 3, 1984, pages 221-227, Springer-Verlag; P. SOKOLOFF et al.: "3H-domperidone binding sites differ in rat striatum and pituitary"**

**NAUNYN-SCHMIEDEBERG'S ARCH. PHARMA-COL., vol. 329, no. 3, mai 1985, pages 236-243, Springer-Verlag; P. SOKOLOFF et al.: "3H-(-)DO 710 discriminates guanine nucleotide sensitive and insensitive dopamine binding sites"**

(73) Titulaire: **SOCIETE D'ETUDES SCIENTIFIOUES ET INDUSTRIELLES DE L'ILE-DE-FRANCE**
**46, Boulevard de Latour-Maubourg**
**F-75340 Paris Cédex 07(FR)**

(72) Inventeur: **Besançon, Denis, Dr.**
**31, boulevard de Latour-Maubourg**
**F-75007 Paris(FR)**
Inventeur: **Costall, Brenda, Dr. Postgraduate School of**
**Studies in Pharmacology University of Bradford**
**Bradford West Yorkshire BD7 IDP(GB)**
Inventeur: **Naylor, Robert J., Dr. Postgraduate School of**
**Studies in Pharmacology University of Bradford**
**Bradford West Yorkshire BD7 IDP(GB)**
Inventeur: **Jenner, Peter, Dr. Institute of Psychiatry**
**De Crespigny Park Denmark Hill**
**Londres SE5 BAF(GB)**
Inventeur: **Marsden, Charles D., Prof. Institute of Psychiatry**
**De Crespigny Park Denmark Hill**
**Londres SE5 BAF(GB)**

Rank Xerox (UK) Business Services

EP 0 210 099 B1

NEUROLOGY & NEUROBIOLOGY, vol. 8B, no. "Catecholamines: Neuropharmacology and central nervous system-theoretical aspects, partie B, 1984, pages 59-72, Alan R. Liss, Inc., New York, US; J.-C. SCHWARTZ et al.: "Biochemical and behavioral identification of discriminant benzamide derivatives: new tools to differentiate subclasses of dopamine receptors"

G.D. BURROWS et al.: "Handbook of studies on schizophrenia", part 2: "Management and research , pages 243-251, Elsevier Science Publishers B.V., Amsterdam, NL; P. SEEMAN: "Dopamine/neuroleptic receptors in schizophrenia"

PHARMACOL. REV., vol. 32, no. 3, 1980, pages 229-313, The American Society for Pharmacology and Experimental Therapeutics; P. SEEMAN: "Brain dopamine receptors"

## Description

La présente invention concerne une nouvelle application du N-(1-allyl-2-pyrrolidinylméthyl) 2-méthoxy 4-amino 5-méthylsulfamoyl benzamide.

Les résultats d'études biochimiques et cliniques ont démontré son efficacité pour soigner la maladie de Parkinson.

Ce composé possède d'intéressantes propriétés. Il présente notamment un effet agoniste dopaminergique indirect particulièrement net, mais ne bloque pas le récepteur dopaminergique post-synaptique. Ceci n'était pas prévisible d'après les résultats habituellement obtenus avec les benzamides, qui sont connus peur bloquer ledit récepteur, voir par exemple la publication Nannyn-Schmiedeberg's Arch. Pharmacolos., 329 (1985), p. 236-243 qui divulgue le fait que le site D4 pent être le mediateur de certains comportements stéréotypes, déclenchés par les agonistes dopaminergiques, qui sont bloqués par ces bemtamides à faible dose.

Les traitements usuels de la maladie de Parkinson visent à stimuler ce récepteur, et l'administration d'un produit potentiellement bloquant était a priori peu indiquée.

Les traitements connus de cette maladie comprennent :
- les anticholinergiques.
- la L-Dopa et ses associations avec des inhibiteurs de la decarboxylase
- les agonistes dopaminergiques vrais : bromocryptine, lergotrile, piribedil, amantadine.

Les autres benzamides, en raison de leur capacité à bloquer le récepteur post-synaptique, étaient considérés classiquement comme responsables de syndrome parkinsonien.

Contrairement à cela, l'étude du composé selon l'invention a permis de révéler ses propriétés dopaminomimétiques indirectes, et son incapacité à bloquer le récepteur postsynaptique.

Cette invention présente donc un grand intérêt. La mise en évidence de l'activité du composé, selon l'invention, a été faite au moyen d'un modèle animal expérimental, ayant une correspondance clinique. Il s'agit de l'étude de l'intoxication par le MPTP (ou 1-méthyl-4-phenyl-1,2, 3, 6-tétrahydropyridine) qui à faible dose détruit sélectivement les neurones dopaminergiques pigmentés, chez le singe comme chez l'homme. Le MPTP reproduit ainsi les signes physiques et biochimiques de la maladie de Parkinson, y compris sa capacité de réponse thérapeutique à la L-Dopa ("MPTP Parkinsonism", British Medical Journal, vol. 289, pages 1401-1402, Novembre 1984). Un protocole particulier permettant d'expérimenter sur le rat, qui n'est pas un modèle usuel de réponse au MPTP, a été mis au point. Il consiste à perfuser pendant 13 jours au niveau de la substancia nigra du rat mâle, la neurotoxine sélective MPTP. Environ 12 heures après le début de la perfusion, apparaissent des effets toxiques, (hypokinésie et bradykinésie) qui persistent pendant les 13 jours, voire même plus longtemps (jusqu'à 40 jours après la fin de la perfusion).

On peut obtenir la quasi suppression de l'hypokinésie et de la bradykinésie, par un traitement levodopa/benserazide. Le remplacement de la L-Dopa par la substance à étudier permet donc une évaluation de l'activité anti-parkinsoniène de cette substance.

Chez le singe marmoset, modèle habituel de ce type d'expérimentation, les mêmes résultats sont obtenus. Or, le composé de l'invention, à des doses de 1 mg/kg/SC produit un antagonisme de l'akinésie induite par le MPTP, de la même façon qu'un traitement par la L-Dopa (100 mg/kg/IP) et le pergolide (0,5 mg/kg/IP), dans les modèles rat et singe.

Les données pharmacologiques ayant trait à l'activité du composé de l'invention sur les récepteurs dopaminergiques ont conduit la demanderesse à des conclusions inattendues.

Les études de "binding" réalisées in-vitro ont démontré que le composé de l'invention agit comme antagoniste dopamine D2 spécifique, avec des effets modérés sur les récepteurs α.

Il diffère des neuroleptiques classiques par son action sur les récepteurs dopaminergiques centraux. Son action anticholinergique centrale est nulle, comme le démontre le test à l'oxotrémorine chez la souris.

Les résultats expérimentaux suivants ont été observés :

1) Seules de très hautes doses (≥80 mg/kg/Ip) peuvent diminuer la motilité spontanée chez la souris.

2) On n'observe aucune anomalie de la coordination ou de la motilité forcée, sur des souris soumises au test de la tige tournante, après l'administration de doses atteignant 80 mg/kg/IP.

3) Aucune catalepsie n'est induite chez le rat, même à des doses de 200 mg/kg/SC.

4) L'antagonisme au comportement de verticalisation (souris) et aux stéréotypies (rats et souris), induïtes par l'apomorphine, ne se produit qu'à très hautes doses du composé, l'effet ne parvenant pas à dépasser le stade partiel pour l'inhibition des stéréotypies.

5) L'antagonisme au vomissement induit chez le chien par l'apomorphine et l'ergot de seigle, ou à l'hypothermie induite chez la souris par l'apomorphine, à des doses inhibitrices-50 de 1 μg/kg/SC, 48 μg/kg/SC et 1,9 mg/kg/IP, démontrent l'effet antidopaminergique puissant sur l'area postrema et l'hypothalamus. Au niveau de l'hypophyse, un effet antidopaminergique puissant se manifeste aussi par l'élévation du taux sérique de prolactine constaté chez le rat mâle, après injection IV d'une très faible dose du produit de l'invention (3 μg/kg). Ces données objectivent l'action antidopaminergique périphérique puissante du composé.

6) A faibles doses le composé potentialise les stéréotypies induites chez le rat par de faibles doses d'apomorphine et les mouvements de mâchonnement ou mordillement induits chez la souris par l'apomorphine.

Les neuroleptiques conventionnels (haloperidol, chlorpromazine) ou même le sulpiride, sont inactifs dans ces conditions.

7) A faible dose, le composé de l'invention potentialise chez le rat, les stéréotypies buccales induites par de faibles doses de d-amphétamine. Aux mêmes doses, les neuroleptiques classiques et le sulpiride, ne potentialisent pas ce comportement.

8) L'injection intrastriatale n'induit pas, contrairement à celle de sulpiride par exemple, ou d'autres benzamides, un comportement d'assymétrie chez le rat dans les déplacements circulaires spontanés, y compris après administration préalable d'apomorphine, même à des doses supérieures à 5 μg.

Ceci est l'indice du fait que le composé ne bloque pas les récepteurs post-synaptiques striataux.

De l'effet activant dopaminomimétique indirect et de l'absence de blocage post-synaptique striatal du composé, la demanderesse a eu l'idée d'un usage possible dans le traitement de la maladie de Parkinson.

Sur la base des résultats expérimentaux animaux, la potentialisation des anti-parkinsoniens traditionnels, ajoutée à la suppression possible des effets secondaires périphériques inhérents à ces agonistes (vomissements, hypotension) fut envisagée en raison du puissant blocage périphérique (dopamine) observé avec les composés benzamides.

Cette hypothèse trouva confirmation dans les investigations pharmacologiques, l'administration du composé de l'invention accroissant l'activité spontanée, comme l'activité induite par l'apomorphine, prouvant ainsi l'effet agoniste de la dopamine (accroissement de l'activité locomotrice, avec des doses variant de 0,001 mg/kg à 40 mg/kg/SC).

Les essais cliniques permettent de souligner l'absence de manifestations extrapyramidales. Les doses thérapeutiques recommandables varient de 10 à 100 mg/jour en une ou plusieurs prises, selon la gravité de la maladie.

**Revendications**

1. Utilisation du N-(1-allyl 2-pyrrolidinylméthyl)-2-méthoxy 4-amino 5-méthylsulfamoyl benzamide et de ses sels pharmacologiquement acceptables, pour la préparation d'un médicament traitant la maladie de Parkinson.

**Claims**

1. Use of N-(1-allyl 2-pyrrolidinylmethyl)-2-methoxy 4-amino 5-methylsulphamoyl benzamide and the pharmacologically acceptable salts thereof, for the preparation of a medicament for treating Parkinson's disease.

**Patentansprüche**

1. Verwendung von N-(1-Allyl-2-pyrrolidinylmethyl)-2-methoxy-4-amino-5-methylsulfamoylbenzamid und seiner pharmakologisch verträglichen Salze zur Herstellung eines Medikaments zur Behandlung der Parkinson-Krankheit.